# EUROPÄISCHE PATENTSCHRIFT

(11) **EP 1 086 717 B1**
(45) Veröffentlichungstag und Bekanntmachung des Hinweises auf die Patenterteilung: **26.05.2004**
(21) Anmeldenummer: 99117905.2
(22) Anmeldetag: 13.09.1999
(51) Int. Cl.: A61M 25/10, A61B 17/12

(54) **Aortale Ballonocclusionskanüle**
Aortic balloon occlusion cannula
Canule aortique à ballonets d'occlusion

(43) Veröffentlichungstag der Anmeldung: 28.03.2001
(73) Patentinhaber: Scheule, Albertus Dr., 72074 Tübingen (DE)
(72) Erfinder: Scheule, Albertus Dr., 72074 Tübingen (DE)
(74) Vertreter: Patentanwälte Rüger, Barthelt & Abel

(56) Entgegenhaltungen:
- WO-A-98/11831
- US-A- 4 731 055
- US-A- 5 312 344
- US-A- 5 458 574

## Beschreibung

Die Erfindung betrifft eine aortale Ballonocclusionskanüle zur Occlusion der Aorta ascendens während herzchirurgischer Eingriffe.

Eine Ballonokklusionskanüle gemäß dem Oberbegriff von Anspruch 1 ist aus der US-A-5 312 344 bekannt.

Ein Problem in der Herzchirurgie ist eine arteriosclerotisch veränderte aufsteigende Hauptschlagader (Aorta ascendens). Diese liegt bei fast allen Patienten mit Herzkranzgefäß-Verkalkung und auch bei vielen Patienten mit Klappenleiden vor. Um bspw. bei einer Bypass-Operation einen Patienten an die extrakorporale Zirkulation (Herz-Lungen-Maschine) anzuschließen, muss eine aortale Kanüle in die Aorta ascendens eingeführt werden. Die Blutzirkulation wird vom Herzen dadurch abgetrennt, dass die Aorta ascendens herzwärts, d. h. proximal mit einer quer angesetzten Metallklemme abgeklemmt wird. Dabei besteht jedoch die Gefahr, dass sich mehr oder weniger große Partikel oder Plaques von der Wandung der Aorta ascendens lösen, die vom Blutstrom vor allem in die Blutgefäße des Kopfes und somit ins Gehirn transportiert werden. Dies hat Embolien zur Folge, die klinisch oft durch neurologische Ausfälle (Hirninfarkt) in Erscheinung treten.

Aus der DE 195 15 933 A1 ist eine aortale Ballonocclusionsperfusions-Kanüle bekannt, die zur Vermeidung einer Risiko behafteten queren Aortenabklemmung während der extrakorporalen Zirkulation bei herzchirurgischen Eingriffen vorgesehen ist. Sie weist eine in einen Katheter einführbare Occlusionskanüle auf, deren Lumen endseitig mit einem dilatierbaren Ballon in Verbindung steht, der es erlaubt, während der Ischämiezeit unter Verzicht der queren Aortenabklemmung die Aorta ascendens durch Ballonocclusion von innen her abzusperren. Eine ähnliche aortale Occlusionskanüle ist auch in der US-A 5 334 142, insbesondere im Zusammenhang mit der kardiopulmonären Wiederbelebung beschrieben.

Diese Ballonocclusionskanülen erlauben es aber nicht anderen Gefahren zu begegnen, die bei einer arteriosclerotischen Aorta ascendens nicht weniger zu Kalkembolien führen können:

Um bei einer Bypass-Operation die Venen-Bypässe an der Aorta annähen zu können, muss die Aorta über eine bestimmte Länge im Bereiche der Nahtstelle tangential mit einer Metallklemme ausgeklemmt werden. Das mit dieser Maßnahme verbundene Embolierisiko ist beträchtlich. Außerdem muss, um das Herz für die Operation ruhig zu stellen, zusätzlich noch ein Kardioplegiemittel in die Aorta ascendens eingeführt werden. Wenn, wie heute gebräuchlich, dazu eine eigene Kardioplegieleitung in die Aorta ascendens eingebracht wird, besteht die Gefahr, dass dabei Plaques abgesprengt werden.

Aufgabe der Erfindung ist es hier abzuhelfen und eine aortale Ballonocclusionskanüle zur Occlusion der Aorta ascendens während chirurgischer Eingriffe zu schaffen, die die Gefahr des Absprengens von Kalkplaques bei verkalkter Aorta ascendens wesentlich verringert und eine schonende Behandlung der Aorta während des chirurgischen Eingriffs gewährleistet.

Zur Lösung dieser Aufgabe weist die erfindungsgemäße Ballonocclusionskanüle die Merkmale des Patentanspruchs 1 auf.

Die neue Aortenkanüle erlaubt es, die Aorta ascendens von innen durch einen dilatierbaren Occlusionsballon abzuklemmen. Zusätzlich dazu trägt die Kanüle einen zweiten Occlusionsballon, der im Abstand von dem ersten Ballon angeordnet ist und es gestattet, einen sich durch den Abstand der beiden Ballons vorgegebenen Bereich von der Perfusion abzutrennen. Dieser Bereich steht dann für das Annähen von Venen-Bypässen zur Verfügung, womit das gefährliche tangentiale Ausklemmen eines Aortenareals vermieden wird. Währenddessen kann die Aortenwurzelperfusion durch eine Leitung herzwärts vollzogen werden, um dadurch die Ischämiezeit zu verkürzen.

Zusätzlich kann die neue Kanüle noch die Funktion der Zuleitung der Herzschutzlösung zu dem Herzen übernehmen und fakultativ auch ein Absaugen ermöglichen. Der Vorteil der neuen Aortenkanüle liegt dabei darin, dass die Aorta im Gegensatz zu dem eingangs erläuterten Stand der Technik nicht von außen her - weder tangential noch quer - abgeklemmt und die Leitung für die Herzschutzlösung nicht extra über einen eigenen weiteren Zugang in die Aorta eingeführt werden muss. Dadurch ist u.a. auch das Operationsfeld übersichtlicher, weil keine weiteren Leitungen und Klemmen das Operationsfeld beeinträchtigen.

Weiterbildungen der neuen Ballonocclusionskanüle sind Gegenstand von Unteransprüchen.

In der Zeichnung ist ein Ausführungsbeispiel des Gegenstandes der Erfindung dargestellt. Es zeigen:
- Fig. 1: eine Ballonocclusionskanüle gemäß der Erfindung in Situ, in einer schematischen Darstellung,
- Fig. 2: die Kanüle nach Fig. 1, geschnitten längs der Linie II-II der Fig. 1, in einer schematischen Darstellung und
- Fig. 3: die Ballonocclusionskanüle nach Fig. 1 im axialen Schnitt, in einer Seitenansicht und in stark vereinfachter Darstellung.

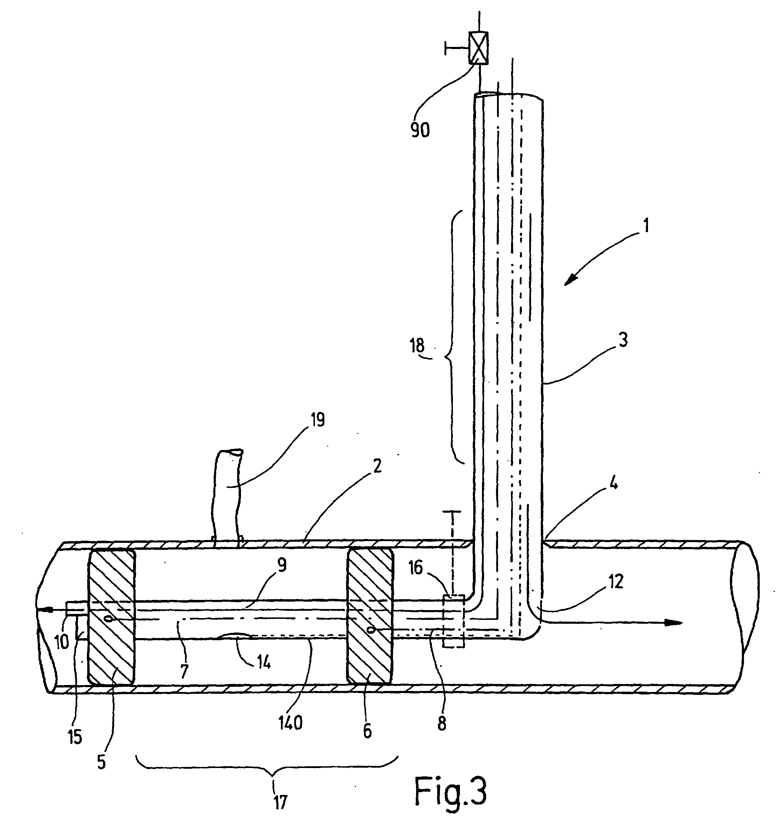

Die allgemein mit 1 bezeichnet aortale Ballonocclusionskanüle dient zur Occlusion der bei 2 angedeuteten Aorta ascendens während herzchirurgischer Eingriffe. Sie weist ein Kanülenrohr 3 auf, das aus einem elastischen Material besteht, welches es erlaubt, das Kanülenrohr 3 unter Anpassung an die erforderlichen Krümmungen in die Aorta ascendens durch eine entsprechende Incision bei 4 (Fig. 3) einzubringen. Das Kanülenrohr 3 kann auch entsprechend der Krümmung der Aorta ascendens vorgeformt sein. Auf das Kanülenrohr 3 sind zwei im Abstand voneinander angeordnete, dilatierbare Occlusionsballons 5 und 6 aufgesetzt, von denen der erste Ballon 5 an dem dem Herzen benachbarten, distalen Ende des Kanülenrohrs 3 angeordnet ist, während der andere Ballon 6 in einem Abstand von ca. 20 bis 30 mm von dem Ballon 5 vorgesehen ist.

Die beiden Occlusionsballons 5, 6 bestehen aus einem elastisch aufweitbaren Kunststoff, bspw. Polyethylen, der über eine ausreichende Formbeständigkeit und Steifigkeit verfügt, um einen sicheren Abschluss der Aorta ascendens 2 zu gewährleisten. Der Durchmesser der beiden Ballons 5, 6 ist an den Innendurchmesser der Aorta ascendens 2 angepasst und liegt in der Größenordnung von ca. 30 mm. Die axiale Länge der beiden Occlusionballons 5, 6 beträgt jeweils etwa 1,5 bis 2 cm oder mehr. Die beiden Occlusionsballons 5, 6 können auf dem Kanülenrohr 3 entweder ortsfest oder gegeneinander verschieblich angeordnet sein, um damit eine Anpassung an die anatomische Gegebenheiten des Einzelfalles zu ermöglichen.

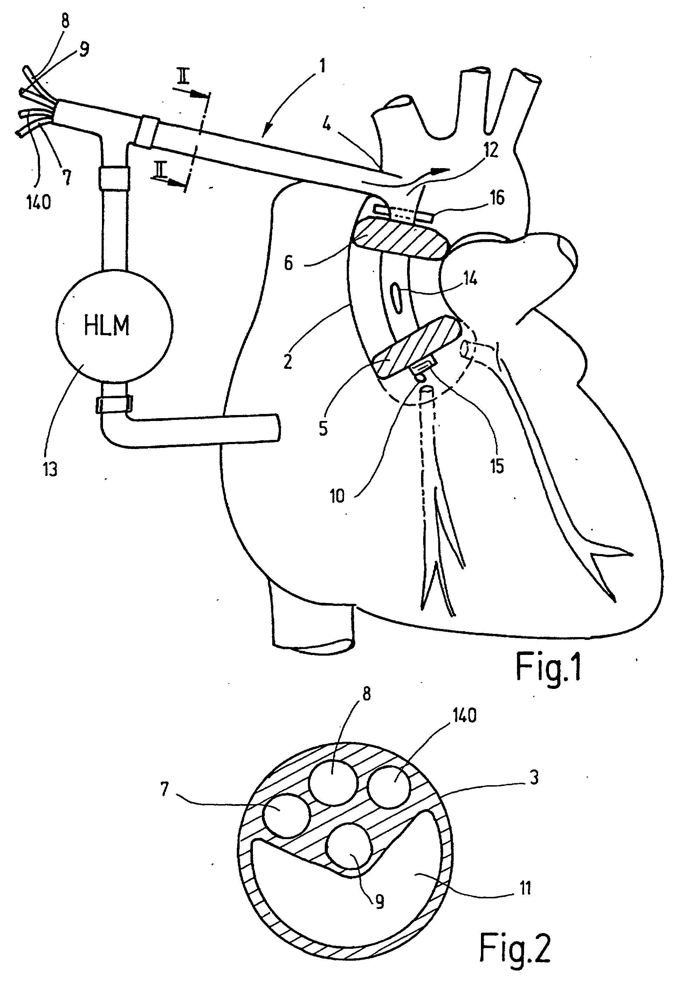

In der Kanüle 1 sind mehrere getrennte Lumina enthalten, die voneinander unabhängige Leitungen bilden und bspw. in der aus Fig. 2 schematisch angedeuteten Weise gegeneinander abgegrenzt sein können:

Ein erstes Lumen 7, dass in Fig. 3 durch eine strichpunktierte Linie angedeutet ist, führt zu dem ersten Occlusionsballon 5 und erlaubt es, diesen über eine geeignete Dilatationsflüssigkeit (physiologische Kochsalzlösung) zu dilatieren. Ein zweites Lumen 8, dass in Fig. 3 durch eine Strich-Doppelpunkt-Linie angedeutet ist, führt zu dem zweiten Occlusionsballon 6 und dient dazu, diesen mittels der entsprechenden Dilatationsflüssigkeit aufzuweiten.

Den durch die Lumina 7, 8 gebildeten Leitungen sind außerhalb des Kanülenrohrs 3 entsprechende Anschlusseinrichtungen für die Dilatationsflüssigkeitsversorgung, Absperrventile und Einrichtungen zugeordnet, um die Occlusionsballons 5, 6 unter Occlusion der Aorta ascendens 2 aufzuweiten und sie wieder in den nicht aufgeweiteten Zustand zurückzuführen. Diese Mittel und Einrichtungen sind im Einzelnen nicht dargestellt. Sie sind bekannt.

Ein drittes Lumen 9 bildet eine die beiden Occlusionballons 5, 6 durchquerende Leitung, die bei 10 auf der dem Herzen zugewandten, distalen Seite des Occlusionsballons 5 mündet. Sie erlaubt es, dem Herzen eine Herzschutzlösung zuzuführen oder bspw. Flüssigkeit aus dem zu dem Occlusionsballon 5 distalen Teil der Aorta ascendens abzusaugen. Die durch das Lumen 9 gebildete Leitung enthält ein Absperrorgan 90, das es gestattet die Zufuhr der Herzschutzlösung bedarfsgemäß zu steuern. Der lichte Durchmesser des Lumens 9 beträgt ca. 3 mm um eine Größenordnung anzugeben.

Von dem Kanülenrohr 3 ist ein weiteres, größeres Lumen 11 umschlossen, das bei in die Aorta 2 eingesetzter Kanüle über eine Öffnung 12 in der Kanülenwand 3 mit dem Lumen des körperseitigen distalen Teils der Aorta ascendens in Verbindung steht. Das Lumen 11 bildet eine Blutleitung, die, wie in Fig. 1 schematisch angedeutet, mit einer Herz-Lungen-Maschine 13 verbunden ist, die extrakorporal die Zirkulation aufrecht erhält. Die Öffnung 12 hat einen Durchmesser von ca. 10 mm oder mehr.

Das Lumen 11 führt außerdem durch die beiden Occlusionsballons 5, 6 hindurch und mündet bei 15 (Fig. 3) auf der distalen Seite des Occlusionsballons 5. Es kann durch einen Schieber 16 abgesperrt werden, der in Fig. 3 nur schematisch angedeutet ist.

Der Flüssigkeitsdurchsatz für die Aortenwurzelperfusion liegt bei ca. 500 bis 800 ml pro Minute.

Schließlich kann die Kanülenwand 3 in dem zwischen den beiden Occlusionsballons 5, 6 liegenden Bereich eine Öffnung 14 aufweisen, die an ein weiteres, getrenntes Lumen in dem Kanülenrohr 3 angeschlossen ist, welches eine in Fig. 3 bei 140 gepunktet angedeutete eigene Leitung bildet, über die Blut aus dem zwischen den beiden Occlusionsballons 5, 6 liegenden Bereich abgesaugt oder dieser mit Flüssigkeit gefüllt werden kann.

Das Kanülenrohr 3 weist einen innerhalb der Aorta ascendens 2 liegenden, im Wesentlichen geraden oder entsprechend der Aorta ascendens gekrümmten Abschnitt 17 auf, von dem ein aus der Aorta herausführender Abschnitt 18 etwa rechtwinklig abgeht. Die Öffnung 12 liegt noch innerhalb der Höhe des geraden Abschnitts 17, d.h. etwas oberhalb der Biegung an der Verbindungsstelle zwischen den beiden Abschnitten 17, 18, um damit sicherzustellen, dass sie bei eingesetzter Kanüle sicher in der Aorta ascendens liegt.

Bei der Benutzung der neuen Kanüle, die auch als aortale Endoclamping-Kanüle mit Doppelballontechnik und integrierter Kardioplegiekanüle bezeichnet werden kann, wird nach dem Einsetzen der Kanüle 1 in die Aorta ascendens 2 zunächst der distale Occlusionsballon 5 dilatiert, während der Schieber 16 geschlossen ist. Damit wird die Aorta ascendens 2 abgesperrt. Über die durch das Lumen 9 gebildete Leitung wird bei 10 eine Kardioplegielösung dem Herzen zugeführt, während der Körperkreislauf über das Lumen 11 und die Öffnung 12 von der Herz-Lungen-Maschine 13 mit Blut versorgt wird. In dieser Zeit werden die herznahen Anastomosen angenäht.

Sodann wird der Schieber 16 geöffnet, womit das Herz über das Lumen 11 wieder mit Blut versorgt wird. Anschließend wird der zweite Occluionsballon 6 aufgeweitet, so dass die Aorta ascendens zusätzlich an dieser Stelle abgesperrt wird. Der zwischen den beiden Ballons 5, 6 begrenzte Bereich der Aorta ascendens 2 wird eröffnet und mit ausgestanzten Anastomosenlöchern versehen, worauf die Anastomosen angenäht werden, von denen eine in Fig. 3 bei 19 angedeutet ist.

Nach Abschluss dieser Maßnahme werden beide Occlusionsballons 5, 6 desouffliert und der Organismus wird von der Herz-Lungen-Maschine abtrainiert. Die Kanüle 1 wird aus der Aorta ascendens 2, gegebenenfalls mit einem sie umgebenden Katheter herausgenommen, worauf die Aorta zugenäht wird.

## Patentansprüche

1. Aortale Ballonocclusions-Kanüle zur Occlusion der Aorta ascendens während herzchirurgischer Eingriffe,
mit einer mehrere voneinander getrennte Lumina enthaltenden Kanüle (1), die zwei im Abstand voneinander angeordnete dilatierbare Occlusionsballons (5,6) trägt, von denen einer dem dem Herzen zugewandten distalen Ende der Kanüle benachbart anzuordnen ist und von denen jeder mit einem eigenen Lumen (7,8) in Verbindung steht, über das er, unabhängig von dem anderen Ballon, dilatierbar ist,
wobei die Kanüle (1) zusätzlich wenigstens ein weiteres Lumen (11) enthält, dadurch gekennzeichet, daß des weitere Lumen (11) auf der distalen Seite des distalen Occlusionsballons (5) und auf der proximalen Seite des anderen proximalen Occlusionsballons (6) mit dem Lumen der Aorta in Verbindung zu bringen ist und zur Verbindung mit einer extrakorporalen Blutversorgungseinrichtung (13) eingerichtet ist, und
das dem auf der distalen Seite des distalen Occlusionsballons (5) mündenden Teil dieses Lumens (11) Absperrmittel (16) zugeordnet sind.

2. Ballonocclusions-Kanüle nach Anspruch 1, **dadurch gekennzeichnet, dass** die beiden Occlusionsballons (5,6) in Achsrichtung gegeneinander verstellbar angeordnet sind.

3. Ballonocclusions-Kanule nach Anspruch 1 oder 2, **dadurch gekennzeichnet, dass** sie ein weiteres getrenntes Lumen (9) enthält, durch das eine auf der distalen Seite des distalen Occlusionsballons (5) mündende Leitung, bspw. für die Kardioplegie und/oder Absaugung gebildet ist.

4. Ballonocclusions-Kanüle nach einem der vorhergehenden Ansprüche, **dadurch gekennzeichnet, dass** die Kanüle (1) in dem Bereich zwischen den beiden Occlusionsballons (5,6) wenigstens eine in diesen Raum mündende Öffnung (14) aufweist, die mit einem eigenen Lumen in der Kanüle (1) in Verbindung steht.

5. Ballonocclusionskanüle nach einem der vorhergehenden Ansprüche, **dadurch gekennzeichnet, dass** sie ein Kanülenrohr (3) aufweist, das im Bereich der beiden Occlusionsballons (5,6) einen im Wesentlichen geraden Abschnitt (17) und einen zweiten Abschnitt (18) aufweist, der bei in die Aorta ascendens (2) eingesetzter Kanüle (1) im Wesentlichen rechtwinklig davon abgeht.

6. Ballonocclusionskanüle nach einem der Ansprüche 1 bis 4, **dadurch gekennzeichnet, dass** sie ein Kanülenrohr (3) aufweist, das zumindest im Bereiche der Occlusionsballons (5,6) einen an den Verlauf der Aorta ascendens angepassten, gekrümmten Abschnitt (17) aufweist.

## Claims

1. Aortal balloon occlusion cannula for occlusion of the ascending aorta during heart surgery,
with a cannula (1) containing several lumina separate from one another and bearing two dilatable occlusion balloons (5, 6) spaced from one another, one of which is to be arranged adjacent to the distal end of the cannula facing the heart and each of which connects to its own lumen (7, 8), via which it is dilatable independently of the other balloon,
wherein the cannula (1) additionally contains at least one further lumen (11), **characterised in that** the further lumen (11) is to be connected to the lumen of the aorta on the distal side of the distal occlusion balloon (5) and on the proximal side of the other proximal occlusion balloon (6) and is fitted for connection to an extracorporeal blood supply unit (13), and
that blocking means (16) are allocated to the part of this lumen (11) opening on the distal side of the distal occlusion balloon (5).

2. Balloon occlusion cannula according to Claim 1, **characterised in that** the two occlusion balloons (5, 6) are arranged to be adjustable relative to one another in axial direction.

3. Balloon occlusion cannula according to Claim 1 or 2, **characterised in that** it contains a further separate lumen (9), through which a pipe opening on the distal side of the distal occlusion balloon (5) is formed, for example, for cardioplegia and/or aspiration.

4. Balloon occlusion cannula according to one of the preceding claims, **characterised in that** in the region between the two occlusion balloons (5, 6), the cannula (1) has at least one opening, which opens into this space and connects to its own lumen in the cannula (1).

5. Balloon occlusion cannula according to one of the preceding claims, **characterised in that** it has a cannula tube (3), which in the region of the two occlusion balloons (5, 6), has an essentially straight section (17) and a second section (18), which, when the cannula (1) is inserted into the ascending aorta (2), projects essentially at right angles therefrom.

6. Balloon occlusion cannula according to one of Claims 1 to 4, **characterised in that** it has a cannula tube (3), which at least in the region of the occlusion balloons (5, 6), has a curved section (17) adapted to the course of the ascending aorta.

## Revendications

1. Canule aortique à ballon d'occlusion pour l'occlusion de l'aorta ascendens pendant des interventions chirurgicales
avec une canule (1) avec plusieurs lumens distincts qui porte deux ballons d'occlusion (5, 6) dilatables, espacés l'un de l'autre, parmi lesquels un est disposé dans le voisinage de l'extrémité distale de la canule tournée vers le coeur, chaque ballon communiquant avec un lumen (7, 8) propre via lequel il peut être gonflé indépendamment de l'autre ballon,
la canule(1) comportant en outre au moins un lumen (11) supplémentaire, **caractérisée par le fait que** le lumen supplémentaire (11), coté distal du ballon d'occlusion (5) distal et coté proximal de l'autre ballon d'occlusion (6) proximal peut être amené en communication avec le lumen de l'aorte et est agencé pour la connexion à un dispositif d'alimentation en sang extra-corporel et **par le fait qu'**un moyen de coupure (16) est associé à la partie dudit lumen (13) qui débouche coté distal du ballon d'occlusion (5) distal.

2. Canule à ballon d'occlusion selon la revendication 1, **caractérisée par le fait que** les deux ballons d'occlusion (5, 6) sont disposés avec possibilité de déplacement l'un par rapport à l'autre dans la direction axiale

3. Canule à ballon d'occlusion selon la revendication 1 ou 2, **caractérisée par le fait qu'**elle comprend un lumen (9) supplémentaire séparé par lequel une conduite débouchant coté distal du ballon d'occlusion distal (5), est formée, par exemple pour la cardioplégie et/ou l'extraction.

4. Canule à ballon d'occlusion selon une des revendications précédentes, **caractérisée par le fait que** la canule (1) dans la partie entre les deux ballons d'occlusion (5, 6), comporte au moins un orifice (14) qui débouche dans cet espace et communique avec un lumen propre dans la canule (1).

5. Canule à ballon d'occlusion selon une des revendications précédentes, **caractérisée par** le fait quelle comporte un tube de canule (3) dans la région des deux ballons d'occlusion (5, 6) présente une portion (17) essentiellement rectiligne et une seconde portion (18) qui, lorsque la canule (1) est insérée dans l'aorta ascendens (2) s'étend essentiellement perpendiculairement à celle-ci.

6. Canule à ballon d'occlusion selon une des revendications 1 à 4, **caractérisée par** le fait quelle comporte un tube de canule (3), qui au moins dans la région des ballons d'occlusion (5, 6), présente une portion (17) courbe adaptée au tracé de l'aorta ascendens.
